# EUROPEAN PATENT APPLICATION

(11) **EP 3 248 532 A1**
(43) Date of publication of application: **29.11.2017**
(21) Application number: 16740039.9
(22) Date of filing: 13.01.2016
(51) Int. Cl.: A61B 1/00

(54) **INSERTION ASSISTANCE TOOL AND MEDICAL DEVICE**

(30) Priority: 20.01.2015 JP 2015008893
(71) Applicant: Olympus Corporation, Tokyo 192-8507 (JP)
(72) Inventor: YOSHIDA, Kazuhiro, Tokyo 192-8507 (JP)
(74) Representative: Schicker, Silvia
(86) International application number: PCT/JP2016/050878
(87) International publication number: WO 2016/117432

(57) **Abstract**

An insertion aid (21) according to the present invention assists in insertion of an insertion member (20) having a rigid distal end part into a paranasal sinus or an opening leading to the paranasal sinus, and includes: a guiding portion (211) having an elongated and curved one end part and configured to guide an insertion route of the insertion member(20); a guided portion (213) including a holding part for holding the insertion member(20) and including a moving part continuous to the holding part and movable along the guiding portion (211); and a support portion (212) for supporting the other end part of the guiding portion (211).

## Description

### Field

The present invention relates to an insertion aid for inserting a member having a rigid distal end into a paranasal sinus. The present invention also relates to a medical device including the insertion aid.

### Background

Conventionally, there are known techniques with which observation and treatment are performed by inserting a medical device such as an endoscope or a treatment tool into a paranasal sinus. The paranasal sinuses include four cavities adjacent to the nasal cavity (frontal sinus, ethmoid sinus, maxillary sinus, and sphenoidal sinus). A practitioner such as a doctor observes and treats the inside of a paranasal sinus by inserting a medical device from an opening through which the nasal cavity leads to each of the cavities.

For example, Patent Literature 1 discloses a rigid guide pipe (guide catheter) to facilitate advancement (insertion) of a medical device into a paranasal sinus. This guide pipe has a distal end part curved at a predetermined angle.

### Citation List

### Patent Literature

Patent Literature 1: JP-T 2009-500051

### Summary

### Technical Problem

For example, a distal end part of an endoscope that is provided with an optical lens has rigidity. This limits the bend radius (minimum bend radius) of the distal end part. Similarly, for example, a distal end part of a treatment tool that is provided with a forceps has rigidity. This limits the bend radius (minimum bend radius) of the distal end part. In a case where a guide pipe employing the technique disclosed in Patent Literature 1 is used, an endoscope or a treatment tool cannot pass through an insertion-side distal end part of the guide pipe when a bend radius of the distal end part of the endoscope or the treatment tool is larger than a curvature radius of the insertion-side distal end part of the guide pipe. As a result, the guide pipe cannot guide the endoscope or the treatment tool into the inside of a paranasal sinus or an opening leading to the paranasal sinus.

The present invention has been made in view of the foregoing, and an object of the invention is to provide an insertion aid and a medical device capable of surely guiding an insertion member into a paranasal sinus or an opening leading to the paranasal sinus, even if the insertion member has a rigid distal end.

### Solution to Problem

In order to solve the above described problem and achieve the object, an insertion aid according to the invention is an insertion aid for assisting in insertion of an insertion member having a rigid distal end part into a paranasal sinus or an opening leading to the paranasal sinus. The insertion aid includes: a guiding portion having an elongated and curved one end part and configured to guide an insertion route of the insertion member; a guided portion including a holding part for holding the insertion member and including a moving part continuous to the holding part and movable along the guiding portion; and a support portion supporting the other end part of the guiding portion.

A medical device according to the invention includes: an insertion member having a rigid distal end part; and an insertion aid for assisting in insertion of the insertion member into a paranasal sinus or an opening leading to the paranasal sinus. The insertion aid includes: a guiding portion having an elongated and curved one end part and configured to guide an insertion route of the insertion member; a guided portion including a holding part for holding the insertion member and including a moving part continuous to the holding part and movable along the guiding portion; and a support portion supporting the other end part of the guiding portion.

### Advantageous Effects of Invention

According to the present invention, it is possible to surely guide an insertion member into a paranasal sinus or an opening leading to the paranasal sinus even if the insertion member has a rigid distal end. Brief Description of Drawings

FIG. 1 is a schematic view illustrating a configuration of a paranasal sinuses observation system including a medical device according to a first embodiment of the present invention.
FIG. 2 is a schematic view illustrating a configuration of an insertion member according to the first embodiment of the present invention.
FIG. 3 is a perspective view schematically illustrating a configuration of the medical device according to the first embodiment of the present invention.
FIG. 4 is a cross-sectional view illustrating configurations of main parts of an insertion aid according to the first embodiment of the present invention.
FIG. 5 is a schematic view illustrating a use situation of the medical device according to the first embodiment of the present invention.
FIG. 6 is a schematic view illustrating a use situation of the medical device according to the first embodiment of the present invention.
FIG. 7 is a schematic view illustrating configurations of main parts of an insertion aid according to a first modification of the first embodiment of the present invention.
FIG. 8 is a schematic view illustrating configurations of main parts of an insertion aid according to a second modification of the first embodiment of the present invention.
FIG. 9 is a schematic view illustrating configurations of main parts of an insertion aid according to a third modification of the first embodiment of the present invention.
FIG. 10 is a perspective view schematically illustrating configurations of main parts of a medical device according to a second embodiment of the present invention.
FIG. 11 is a schematic view illustrating configurations of main parts of an insertion aid according to the second embodiment of the present invention.
FIG. 12 is a perspective view schematically illustrating a configuration of a medical device according to a third embodiment of the present invention.
FIG. 13 is a perspective view schematically illustrating a configuration of a medical device according to a fourth embodiment of the present invention.
FIG. 14 is a perspective view schematically illustrating configurations of main parts of an insertion aid according to the fourth embodiment of the present invention.
FIG. 15 is a schematic view illustrating a relationship between a slider and piece parts of the insertion aid according to the fourth embodiment of the present invention.
FIG. 16 is a schematic view illustrating a holding condition of a holding part of a regulating member according to the fourth embodiment of the present invention.
FIG. 17 is a schematic view illustrating a relationship between a moving part and the piece parts of the regulating member according to the fourth embodiment of the present invention.
FIG. 18 is a schematic view illustrating a holding condition of the holding part of the regulating member according to the fourth embodiment of the present invention.
FIG. 19 is a perspective view schematically illustrating a configuration of a medical device according to a fifth embodiment of the present invention.
FIG. 20 is a perspective view schematically illustrating configurations of main parts of an insertion aid according to the fifth embodiment of the present invention.
FIG. 21 is a perspective view schematically illustrating configurations of main parts of an insertion aid according to the fifth embodiment of the present invention.
FIG. 22 is a schematic view illustrating a holding condition of a holding part of a regulating member according to the fifth embodiment of the present invention.
FIG. 23 is a schematic view illustrating a holding condition of the holding part of the regulating member according to the fifth embodiment of the present invention.
FIG. 24 is a schematic view illustrating an exemplary arrangement of an insertion member according to a sixth embodiment of the present invention.
FIG. 25 is a schematic view illustrating an exemplary configuration of a distal end of a guide sheath according to a seventh embodiment of the present invention.
FIG. 26 is a top view as seen from an arrow A direction illustrated in FIG. 25.

### Description of Embodiments

Hereinafter, modes for carrying out the present invention (hereinafter referred to as "embodiment(s)") will be described with reference to the accompanying drawings.

### (First Embodiment)

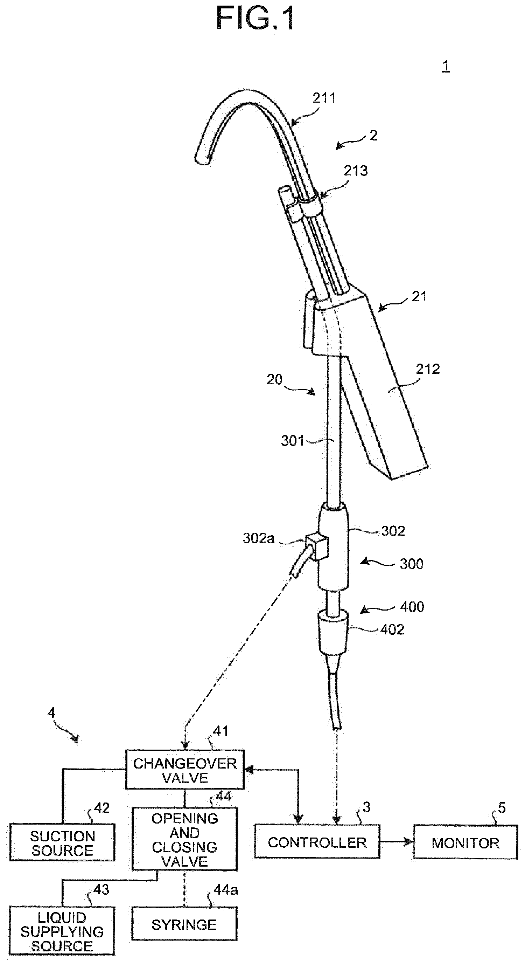

FIG. 1 is a schematic view illustrating a configuration of a paranasal sinuses observation system 1 including a medical device 2 according to a first embodiment of the present invention. The paranasal sinuses observation system 1 illustrated in FIG. 1 is a system for observing the paranasal sinuses, and includes the medical device 2 capable of obtaining an image of the inside of the paranasal sinuses, a controller 3, a liquid supply and suction operating unit 4, and a monitor 5. The controller 3 controls the entire paranasal sinuses observation system 1 including the medical device 2, and generates an image on the basis of a signal obtained by the medical device 2. The liquid supply and suction operating unit 4 controls a liquid sending operation and a suction operation performed by the medical device 2. The monitor 5 displays the image generated by the controller 3.

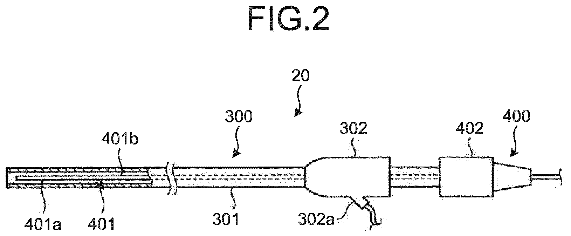

The medical device 2 includes an insertion member 20 and an insertion aid 21. FIG. 2 is a schematic view illustrating a configuration of the insertion member 20. The insertion member 20 includes a sheath portion 300 and an endoscope 400. The endoscope 400 is implemented using a scanning type endoscope having a plurality of fibers. Instead of the scanning type endoscope, any types of endoscopes such as an endoscope including an image sensor such as a CCD or a CMOS may be used. However, it is preferable to use the scanning type endoscope in terms of reducing an outer diameter of the sheath portion 300. In the first embodiment, the endoscope 400 is a scanning type endoscope. A fiber group is inserted into the endoscope 400. The fiber group includes a fiber for illumination and a plurality of fibers for receiving light, each of which is connected to the controller 3. The fiber for illumination emits illumination light from a distal end thereof, and the plurality of fibers for receiving light receives light reflected and scattered by illumination light.

The sheath portion 300 has a tubular guide sheath 301 and a sheath holding part 302. The guide sheath 301 is elongated and has a small diameter and flexibility. The sheath holding part 302 holds a proximal end of the guide sheath 301. The sheath holding part 302 is provided with a connection portion 302a connecting to the liquid supply and suction operating unit 4 via a tube. Hereinafter, the guide sheath 301 will be described as having a cylindrical shape with a uniform outer diameter.

The endoscope 400 has an insertion portion 401 and an operation portion 402. The insertion portion 401 is inserted into the guide sheath 301. The fiber for illumination and the plurality of fibers for receiving light are inserted into the insertion portion 401. At least a distal end of the insertion portion 401 is inserted into the paranasal sinus. The operation portion 402 holds a proximal end of the insertion portion 401, and is operable in a direction approaching or away from the sheath holding part 302.

Main parts of the insertion portion 401 are formed by connecting, in order from a side of the distal end, a distal end rigid part 401a having rigidity, and a flexible tube part 401b. The flexible tube part 401b is located at a rear end of the distal end rigid part 401a and extends to the operation portion 402, and is elongated and has a small diameter and flexibility. The distal end rigid part 401a is provided with an illumination window, an actuator, and the like. According to the first embodiment, an observation optical system is formed with the illumination window, the actuator, the fiber for illumination, and the plurality of fibers for receiving light.

In the insertion member 20, the insertion portion 401 (the distal end rigid part 401a) can be extended from a distal end of the guide sheath 301 by moving the operation portion 402 close to the sheath holding part 302, and the insertion portion 401 (the distal end rigid part 401a) can be housed inside the guide sheath 301 by moving the operation portion 402 away from the sheath holding part 302.

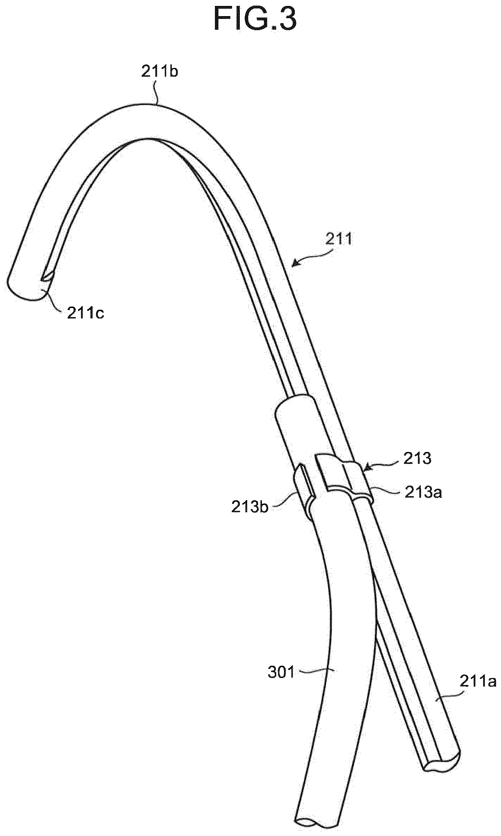

FIG. 3 is a perspective view schematically illustrating a configuration of the medical device 2. As illustrated in FIGS. 1 and 3, the insertion aid 21 assists an insertion of the insertion member 20 when the insertion member is inserted into a paranasal sinus or an opening leading to the paranasal sinus. The insertion aid 21 includes a guiding portion 211, a grip portion 212 (support portion), and a regulating member 213 (guided portion). The guiding portion 211 is elongated and guides an insertion route of the insertion member 20 into a paranasal sinus. The grip portion 212 is provided at a proximal end of the guiding portion 211, supports the guiding portion 211, and is gripped by a practitioner or the like. The regulating member 213 can hold the sheath portion 300, and is attached to the guiding portion 211 and movable along the guiding portion 211.

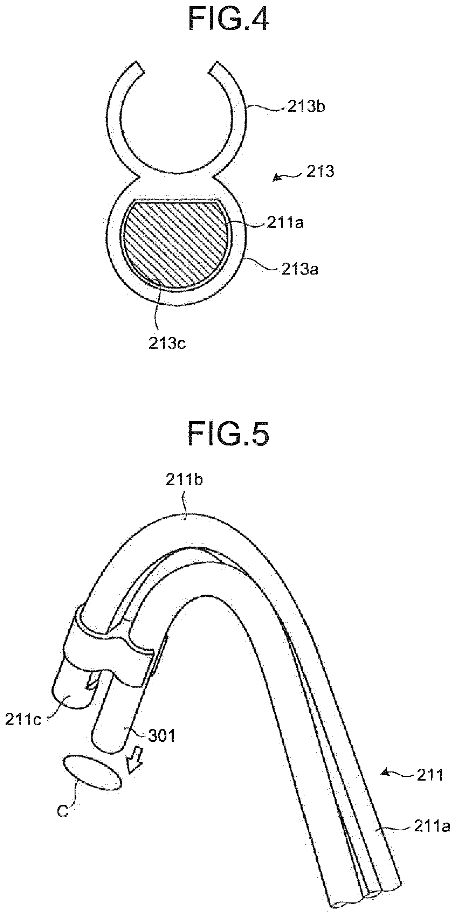

FIG. 4 is a cross-sectional view illustrating configurations of main parts of the insertion aid 21, the cross-sectional view illustrating a cutting surface that is a plane including the regulating member 213 and perpendicular to an extending direction of the guiding portion 211. FIG 4 is a cross-sectional view of a first guiding part 211a, as an example of a cross section of the guiding portion 211, the cross-sectional view illustrating a cutting surface that is a plane perpendicular to an extending direction of the first guiding part 211a. As illustrated in FIG. 4, the guiding portion 211 is formed by curving a columnar member having a D-shaped cross sectional shape. The columnar member has, for example, a shape obtained by cutting out a cylindrical member along a longitudinal direction thereof, the columnar member having a planar section formed by a cutout (D cut). A tubular member may be used instead of the columnar member.

The guiding portion 211 includes the first guiding part 211a, a second guiding part 211b, and a convex part 211c. The first guiding part 211a extends from the grip portion 212 in a substantially linear fashion. The second guiding part 211b is curved at a predetermined curvature radius from an end that is different from an end of the first guiding part 211a connecting to the grip portion 212. The convex part 211c is provided at an end that is different from an end of the second guiding part 211b connecting to the first guiding part 211a, the convex part 211c projecting from a D-cut surface (planar section). A curvature radius of the second guiding part 211b is decided according to a paranasal sinus (frontal sinus, ethmoid sinus, maxillary sinus, and sphenoidal sinus) to which the insertion member 20 is guided. The second guiding part 211b includes a shape curved continuously at different curvature radii.

The guiding portion 211 is preferably formed using a malleable material. Examples of the malleable material include a ductile metal (e.g., aluminum and silver), an alloy of the ductile metal, and a deformable resin.

The regulating member 213 includes a moving part 213a and a holding part 213b. The moving part 213a is attached to and movable along the guiding portion 211. The holding part 213b can hold the sheath portion 300 (guide sheath 301). In the moving part 213a, there is formed an insertion through hole 213c having a D-shaped cross-section corresponding to the cross-section of the guiding portion 211. In the first embodiment, the holding part 213b has a C-shape as seen in a plan view (refer to FIG. 4). Alternatively, the holding part 213b may have an annular shape (O-shape) as long as the holding part 213b is capable of holding the guide sheath 301.

The regulating member 213 can abut a step formed between the guiding portion 211 and the grip portion 212, at the moving part 213a, as well as the convex part 211c. Thus, a moving range of the regulating member 213 is limited. That is, the regulating member 213 is movable between an end of the guiding portion 211 on a side of the grip portion 212 and the convex part 211c.

The cross-sectional shape of the guiding portion 211 and a hole (opening) of the insertion through hole 213c into which the guiding portion 211 is inserted are of a D-shape such that the shapes correspond to each other. These shapes thus prevent the regulating member 213 from rotating around the guiding portion 211.

The regulating member 213 moves according to the movement of the guide sheath 301. Therefore, a frictional resistance between the moving part 213a and the guiding portion 211 is preferably smaller than a frictional resistance between the holding part 213b and the guide sheath 301. To move the regulating member 213 according to the movement of the guide sheath 301, the frictional resistance may be adjusted by a known technique, for example, by applying surface treatment to the guiding portion 211.

The controller 3 is implemented using a central processing unit (CPU) having arithmetic and control functions, various arithmetic circuits, or the like. The controller 3 controls the entire paranasal sinuses observation system 1 including the medical device 2 and generates an image on the basis of a signal obtained by the endoscope 400. Specifically, the controller 3 generates the image on the basis of a signal based on light obtained via the plurality of fibers for receiving light. The signal based on light may be a signal obtained by photoelectrically converting light using the operation portion 402 or the like or a signal obtained by photoelectrically converting light guided by the controller 3 via the plurality of fibers for receiving light.

Furthermore, the controller 3 outputs, to the liquid supply and suction operating unit 4, a control signal for controlling a liquid sending operation and a suction operation. The controller 3 outputs display information including a generated image to the monitor 5, and causes the monitor 5 to display the display information.

Note the controller 3 may have a light source emitting illumination light to be supplied to the fiber for illumination. Alternatively, the controller 3 may guide illumination light from an external light source and supplies the illumination light to the fiber for illumination.

The liquid supply and suction operating unit 4 has a changeover valve 41, a suction source 42, a liquid supplying source 43, and an opening and closing valve 44. The changeover valve 41 is implemented using, for example, a three-way cock, and connects with an end of a tube extending from the connection portion 302a. Furthermore, the changeover valve 41 connects to the suction source 42 at a stopper cock different from the stopper cock connecting with the tube of the three-way cock. Meanwhile, the changeover valve 41 connects to the liquid supplying source 43 via the opening and closing valve 44 at an stopper cock different from the stopper cocks connecting to the tube of the three-way cock and the suction source 42. The liquid supply and suction operating unit 4 selectively switches between the suction source 42 and the liquid supplying source 43 by an operation of the changeover valve 41, thereby supplying liquid to or sucking liquid from the inside of a nasal cavity or a paranasal sinus via the sheath portion 300.

The suction source 42 is implemented using, for example, a suction pipe. A suction device installed in an operation room may be used as the suction source 42. For example, a sticky substance existing around an affected area in the paranasal sinus and the nasal cavity can be removed by actuating the suction source 42. In addition, when the affected area and a surrounding area thereof are cleaned with normal saline, the cleaning liquid can be removed together with the sticky substance by actuating the suction source 42.

The liquid supplying source 43 supplies normal saline for cleaning, for example, the affected area in the paranasal sinus. Furthermore, the liquid supplying source 43 may be configured to supply a medical agent to treat the affected area. The medical agent includes mainly a steroid and an antibacterial agent. The medical agent may contain a temperature responsive gel or the like that increases viscosity thereof when the temperature of the gel reaches around body temperature. In such a case, when the medical agent is administered to the affected area, the body temperature of the patient increases the viscosity of the temperature responsive gel, thus preventing the medical agent from smoothly flowing from the affected area. This prolongs a stagnation time of the medical agent and thus enables the medical agent to be retained in the affected area. The liquid supplied from the liquid supplying source 43 is selectable as needed.

The opening and closing valve 44 is implemented using, for example, the three-way cock. The opening and closing valve 44 connects to the changeover valve 41 and the liquid supplying source 43 at two of the three stopper cocks, and connects to a syringe 44a for medication. The changeover valve 41 and the opening and closing valve 44 may be electromagnetically operated by a flip of a switch (not illustrated) connected to the controller 3. Alternatively, the changeover valve 41 and the opening and closing valve 44 may be switched on or off manually.

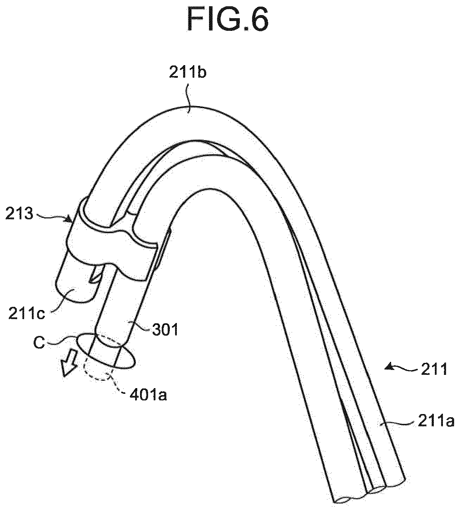

Reference will now be made to a method of inserting the insertion member 20 into an opening of the paranasal sinus in the paranasal sinuses observation system 1 having the configuration described above. FIGS. 5 and 6 are schematic views illustrating a use situation of the medical device 2, and illustrating a method of inserting the insertion member 20 into the opening of the paranasal sinus.

First, as illustrated in FIGS. 1 and 3, the regulating member 213 holds the insertion member 20. Specifically, the holding part 213b holds the guide sheath 301. In this state, the distal end of the guiding portion 211 is moved close to the opening of the paranasal sinus (opening C illustrated in FIG. 5). To cause the distal end of the guiding portion 211 to access the opening C of the paranasal sinus, positions of the distal end of the guiding portion 211 and the opening C of the paranasal sinus may be checked using an image obtained by the endoscope 400 or an image obtained by another endoscope.

The insertion member 20 (guide sheath 301) is moved to a distal end side of the guiding portion 211 while the distal end of the guiding portion 211 is moved close to the opening C of the paranasal sinus (refer to FIG. 5). At this time, the moving part 213a moves to the side of the distal end along the guiding portion 211 according to the movement of the guide sheath 301. By this operation, the distal end of the guide sheath 301 is moved to a position facing the opening C.

Then, the practitioner moves the operation portion 402 close to the sheath holding part 302, thereby extending the insertion portion 401 from the distal end of the guide sheath 301 (refer to FIG. 6). At this time, the insertion portion 401 extending from the distal end of the guide sheath 301 is inserted into the paranasal sinus from the opening C.

The liquid sending operation or the suction operation may be performed via the connection portion 302a before the insertion portion 401 is extended from the distal end of the guide sheath 301. Pus or the like remaining at the opening of the paranasal sinus is cleaned or pus or the like remaining inside the paranasal sinus is sucked by the liquid sending operation or the suction operation. Thus, insertability of the insertion portion 401 can be improved.

According to the first embodiment of the present invention described above, the insertion aid 21 includes the guiding portion 211 and the regulating member 213. The guiding portion 211 is elongated and guides a traveling direction of the sheath portion 300 of the insertion member 20. The regulating member 213 can hold the sheath portion 300, and is attached to the guiding portion 211 and slidable along the guiding portion 211. Using the insertion aid 21, a distal end of the guiding portion 211 is arranged in the vicinity of the opening C of the paranasal sinus. Then, the regulating member 213 holding the sheath portion 300 is slid along the guiding portion 211. Thus, the distal end of the sheath portion 300 is arranged in the vicinity of the opening C of the paranasal sinus. With this configuration, the insertion member 20 can be surely guided regardless of the minimum bend radius defined by the hard length of the distal end rigid part 401a of the insertion portion 401.

Furthermore, in the first embodiment described above, the cross-sectional shape of the guiding portion 211 and the hole (opening) of the insertion through hole 213c into which the guiding portion 211 is inserted are of a D-shape. These shapes prevent the regulating member 213 from rotating around the guiding portion 211. The guide sheath 301 can be guided without interference with obstacles in the nasal cavity by adjusting the orientation of the D-cut surface so as to avoid the obstacles in the nasal cavity.

In the first embodiment described above, the guide sheath 301 is arranged in the vicinity of the opening C of the paranasal sinus. However, the insertion portion 401 may be extended from the distal end of the guide sheath 301 after the guide sheath 301 is inserted into the paranasal sinus. Furthermore, a treatment tool having a rigid distal end part such as a biopsy forceps may be inserted into the guide sheath 301. Furthermore, using the guide sheath 301 alone without the endoscope 400 as the insertion member, a liquid sending process or a suction process may be performed. Alternatively, the endoscope 400 alone without the sheath portion 300 may be used as the insertion member. In a case where the guide sheath 301 is inserted into the opening C, the distal end of the guide sheath 301 preferably has rigidity or hardness of the distal end is preferably higher than those of the other parts, from a viewpoint of facilitating the insertion into the opening C. Furthermore, in a case where the endoscope 400 alone is used as an insertion member, the liquid supply and suction operating unit 4 is connected to the operation portion 402.

### (First Modification of First Embodiment)

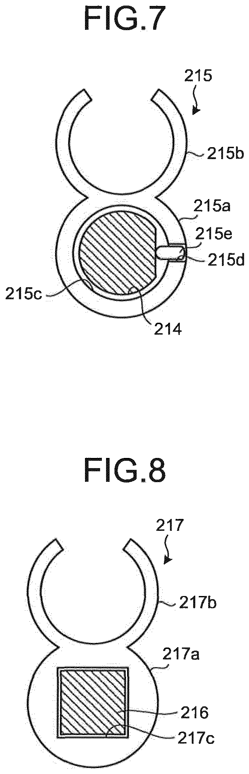

FIG. 7 is a schematic view illustrating configurations of main parts of an insertion aid according to a first modification of the first embodiment of the present invention. FIG. 7 is a cross-sectional view corresponding to the cross-sectional view illustrated in FIG. 4, the cross-sectional view illustrating a cutting surface that is a plane including a regulating member 215 and perpendicular to an extending direction of a guiding portion 214. In the first embodiment, regarding the rotation preventing portion, the hole (opening) of the insertion through hole 213c of the regulating member 213 is of a D-shape to restrict the rotation around the guiding portion 211. However, in the first modification, the rotation is restricted by a screw.

An insertion aid according to the first modification includes a guiding portion 214, a grip portion 212 described above, and a regulating member 215. The guiding portion 214 is elongated and guides a traveling direction of a sheath portion 300 of an insertion member 20. The regulating member 215 can hold the sheath portion 300, and is attached to the guiding portion 214 and movable along the guiding portion 214. As with the guiding portion 211 described above, the guiding portion 214 has a D-shaped cross section perpendicular to an extending direction.

The regulating member 215 includes a moving part 215a and a holding part 215b. The moving part 215a is attached to the guiding portion 214 and movable along the guiding portion 214. The holding part 215b can hold the sheath portion 300 (guide sheath 301). In the moving part 215a, there are formed an insertion through hole 215c having a circular opening corresponding to a circumscribed circle circumscribing the outer periphery of the guiding portion 214 and a screw hole 215d communicating between the insertion through hole 215c and the outside.

A screw 215e is inserted into the screw hole 215d while the guiding portion 214 is inserted into the regulating member 215. In this state, the screw 215e abuts on the D-cut surface of the guiding portion 214. This can restrict the rotation of the regulating member 215 around the guiding portion 214. Instead of the D cut, the guiding portion 214 may have a formed groove extending in a longitudinal direction of the guiding portion 214 and capable of housing a distal end of the screw 215e.

### (Second Modification of First Embodiment)

FIG. 8 is a schematic view illustrating configurations of main parts of an insertion aid according to a second modification of the first embodiment of the present invention. FIG. 8 is a cross-sectional view corresponding to the cross-sectional view illustrated in FIG. 4, the cross-sectional view illustrating a cutting surface that is a plane including a regulating member 217 and perpendicular to an extending direction of a guiding portion 216. In the first embodiment described above, regarding the rotation preventing portion, the cross-sectional shape of the guiding portion 211 and the hole (opening) of the insertion through hole 213c of the regulating member 213 are of a D-shape to restrict the rotation around the guiding portion 211. However, in the second modification, the rotation is restricted by making a hole of an insertion through hole rectangular.

The insertion aid according to the second modification includes a guiding portion 216, a grip portion 212 described above, and a regulating member 217. The guiding portion 216 is elongated and guides a traveling direction of a sheath portion 300 of an insertion member 20. The regulating member 217 can hold the sheath portion 300, and is attached to the guiding portion 216 and slidable along the guiding portion 216. The guiding portion 216 has a rectangular cross section perpendicular to an extending direction.

The regulating member 217 includes a sliding part 217a and a holding part 217b. The sliding part 217a is attached to the guiding portion 216 and slidable along the guiding portion 216. The holding part 217b can hold the sheath portion 300 (guide sheath 301). In the sliding part 217a, there is formed an insertion through hole 217c having a rectangular opening corresponding to the cross-sectional shape of the guiding portion 216.

According to the second modification, the insertion through hole 217c has a rectangular shape corresponding to a cross-sectional shape of the guiding portion 216 while the guiding portion 216 is inserted into the regulating member 217. This can restrict the rotation of the regulating member 217 around the guiding portion 216.

In the second modification, a disposing position of the sheath portion 300 with respect to the guiding portion 216 can be changed by changing an orientation of the regulating member 217 with respect to the guiding portion 216 (angle in a circumferential direction with respect to the guiding portion 216). With a polygon of a pentagon or more, the disposing position can be more finely adjusted.

### (Third Modification of First Embodiment)

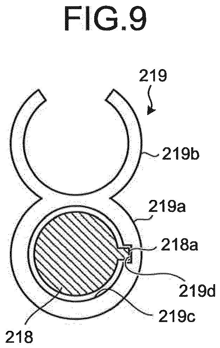

FIG. 9 is a schematic view illustrating configurations of main parts of an insertion aid according to a third modification of the first embodiment of the present invention. FIG. 9 is a cross-sectional view corresponding to the cross-sectional view illustrated in FIG. 4, the cross-sectional view illustrating a cutting surface that is a plane including a regulating member 219 and perpendicular to an extending direction of a guiding portion 218. In the first embodiment, regarding the rotation preventing portion, the hole (opening) of the insertion through hole 213c of the regulating member 213 is of a D-shape to restrict the rotation around the guiding portion 211. However, in the third modification, the rotation is restricted by providing a projection on a guiding portion 218 having a circular cross-sectional shape.

The insertion aid according to the third modification includes a guiding portion 218, a grip portion 212 described above, and a regulating member 219. The guiding portion 218 is elongated and guides a traveling direction of a sheath portion 300 of an insertion member 20. The regulating member 219 can hold the sheath portion 300, and is attached to the guiding portion 218 and slidable along the guiding portion 218. The guiding portion 218 has a substantially circular cross section perpendicular to an extending direction, and has a projection 218a extending along a longitudinal direction of the guiding portion 218 and projecting in a radial direction.

The regulating member 219 includes a sliding part 219a and a holding part 219b. The sliding part 219a is attached to the guiding portion 218, and slidable along the guiding portion 218. The holding part 219b can hold the sheath portion 300 (guide sheath 301). In the sliding part 219a, there are formed an insertion through hole 219c having a substantially circular opening corresponding to a circumscribed circle circumscribing the outer periphery of the guiding portion 218 and a concave part 219d formed so as to correspond to the projection 218a.

The projection 218a of the guiding portion 218 is housed in the concave part 219d while the guiding portion 218 is inserted into the regulating member 219. This can restrict the rotation of the regulating member 219 around the guiding portion 218.

### (Second Embodiment)

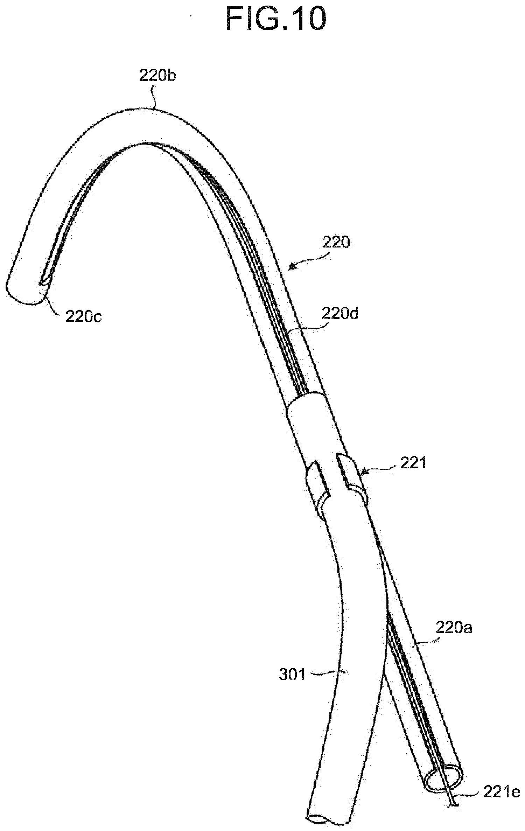

Next, a second embodiment of the present invention will be described. FIG. 10 is a perspective view schematically illustrating configurations of main parts of a medical device according to the second embodiment. The same reference signs are used to designate the same elements as those in the above-described embodiment. In the first embodiment described above, the moving part 213a of the regulating member 213 is attached to the outer periphery of the guiding portion 211. However, in the second embodiment, a guiding portion 220 movably houses a part of a regulating member 221.

As illustrated in FIG. 10, an insertion aid includes a guiding portion 220, a grip portion 212 (refer to FIG. 1), and a regulating member 221. The guiding portion 220 is elongated and guides a traveling direction of an insertion member 20. The grip portion 212 is provided at a proximal end of the guiding portion 220 and gripped by a practitioner or the like. The regulating member 221 can hold the sheath portion 300 and slide along the guiding portion 220.

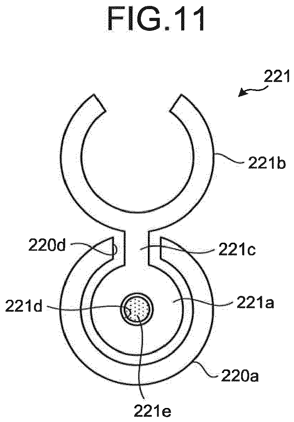

FIG. 11 is a schematic view illustrating configurations of main parts of the insertion aid according to the second embodiment, the schematic view illustrating a relationship between the guiding portion 220 and the regulating member 221, as seen from a longitudinal direction. The guiding portion 220 is formed by curving a member having a substantially cylindrical shape cut out along the longitudinal direction. The guiding portion 220 includes a first guiding part 220a, a second guiding part 220b, and a convex part 220c. The first guiding part 220a extends from the grip portion 212 in a substantially linear fashion. The second guiding part 220b extends from an end that is different from an end of the first guiding part 220a connecting to the grip portion 212 while being curved at a predetermined curvature radius. The convex part 220c is provided at an end that is different from an end of the second guiding part 220b connecting to the first guiding part 220a, the convex part 220c projecting from a cutout surface. Furthermore, in the guiding portion 220, there is formed a slit 220d cut out along a longitudinal direction. A curvature radius of the second guiding part 220b is determined according to a position of a paranasal sinus to which the insertion member 20 is guided, as with the second guiding part 211b described above. The guiding portion 220 is formed using similar materials to those used for the guiding portion 211 described above.

The regulating member 221 includes a moving part 221a, a holding part 221b, and a coupling part 221c. The moving part 221a is housed in the guiding portion 220 and movable along the guiding portion 220. The holding part 221b can hold the sheath portion 300 (guide sheath 301). The coupling part 221c couples the moving part 221a and the holding part 221b. In the moving part 221a, an insertion through hole 221d having a circular opening is formed.

Furthermore, the inside of the guiding portion 220 is provided with a guide wire 221e that is inserted into the insertion through hole 221d. The guide wire 221e may not be provided as long as the guiding portion 220 alone can guide the movement of the regulating member 221.

The regulating member 221 can abut on the grip portion 212 and the convex part 220c at both ends of the guiding portion 220. Thus, a moving range of the regulating member 221 is limited. That is, the regulating member 221 can move between an end of the guiding portion 220 on the side of the grip portion 212 and the convex part 220c. The convex part 220c may not be provided as long as the movement of the regulating member 221 can be restricted, as in a case where the distal end part of the guiding portion 220 is blocked or in a case where the slit 220d does not extend to the distal end part of the guiding portion 220.

The regulating member 221 moves according to the movement of the guide sheath 301. Therefore, a frictional resistance between the moving part 221a and the guiding portion 220 is preferably smaller than a frictional resistance between the holding part 221b and the guide sheath 301.

Furthermore, in the regulating member 221, with the moving part 221a housed in the guiding portion 220, the coupling part 221c engages with the slit 220d (refer to FIG. 11). Therefore, the rotation of the regulating member 221 around the guiding portion 220 can be prevented (rotation preventing portion).

According to the second embodiment of the present invention described above, the insertion aid includes the guiding portion 220 and the regulating member 221. The guiding portion 220 is elongated and guides a traveling direction of the sheath portion 300 of the insertion member 20. The regulating member 221 can hold the sheath portion 300, and is housed in the guiding portion 220 and slidable along the guiding portion 220. Using the insertion aid, a distal end of the guiding portion 220 is arranged in the vicinity of an opening C of a paranasal sinus. Then, the regulating member 221 holding the sheath portion 300 is slid along the guiding portion 220. Thus, the distal end of the sheath portion 300 is arranged in the vicinity of the opening C of the paranasal sinus. With this configuration, the insertion member 20 can be surely guided regardless of the minimum bend radius defined by the hard length of a distal end rigid part 401a of an insertion portion 401.

Furthermore, according to the second embodiment described above, the moving part 221a is housed in the guiding portion 220. With this configuration, further downsizing can be achieved as compared to a configuration, such as the one in which the holding part 213b of the regulating member 213 holds the outer periphery of the guiding portion 211, according to the first embodiment described above.

### (Third Embodiment)

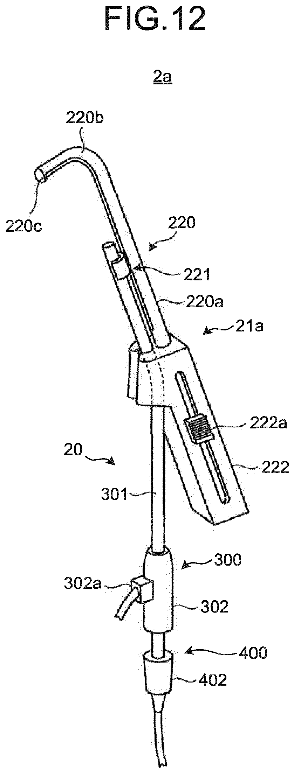

Next, a third embodiment of the present invention will be described. FIG. 12 is a perspective view schematically illustrating a configuration of a medical device 2a according to the third embodiment. The same reference signs are used to designate the same elements as those in the above-described embodiments. In the second embodiment described above, the regulating member 221 moves according to the movement of the guide sheath 301. However, in the third embodiment, a regulating member 221 moves according to the movement of a slider 222a provided at a grip portion 222.

As illustrated in FIG. 12, the medical device 2a includes an insertion member 20 described above and an insertion aid 21a. The insertion aid 21a includes a guiding portion 220, the grip portion 222, and the regulating member 221. The guiding portion 220 is elongated and guides a traveling direction of a sheath portion 300 of the insertion member 20. The grip portion 222 is provided at a proximal end of the guiding portion 220 and gripped by a practitioner or the like. The regulating member 221 can hold the sheath portion 300 and move along the guiding portion 220.

As described above, the guiding portion 220 is formed by curving a tubular member having a substantially cylindrical shape cut out along a longitudinal direction. Furthermore, the regulating member 221 includes a moving part 221a, a holding part 221b, and a coupling part 221c described above. In the third embodiment, the insertion through hole 221d and the guide wire 221e described above are not included.

The grip portion 222 is provided with the slider 222a that can move substantially parallel to an extending direction of a first guiding part 220a.

A flexible connecting wire (not illustrated) connecting the moving part 221a and the slider 222a is provided in the guiding portion 220 and the grip portion 222. Therefore, when the slider 222a is moved, the regulating member 221 moves in conjunction with the movement of the slider 222a. At this time, the insertion member 20 also moves according to the movement of the regulating member 221.

According to the third embodiment of the present invention described above, the insertion aid 21a includes the guiding portion 220, the regulating member 221, and the slider 222a. The guiding portion 220 is elongated and guides a traveling direction of the sheath portion 300 of the insertion member 20. The regulating member 221 can hold the sheath portion 300, and is housed in the guiding portion 220 and slidable along the guiding portion 220. The slider 222a operates the movement of the regulating member 221. Using the insertion aid 21a, a distal end of the guiding portion 220 is arranged in the vicinity of an opening C of a paranasal sinus. Then, the regulating member 221 holding the sheath portion 300 is slid along the guiding portion 220. Thus, the distal end of the sheath portion 300 is arranged in the vicinity of the opening C of the paranasal sinus. With this configuration, the insertion member 20 can be surely guided regardless of the minimum bend radius defined by the hard length of the distal end rigid part 401a of the insertion portion 401.

Furthermore, according to the third embodiment described above, the regulating member 221 is moved by the operation of the slider 222a. With this configuration, there is no need to consider a frictional resistance of the regulating member 221 against the guiding portion 220 and a frictional resistance against the guide sheath 301. Thus, this further facilitates design of a medical device, as compared to a configuration such as the one in which the regulating member 221 is moved in conjunction with the movement of the guide sheath 301, according to the second embodiment described above.

### (Fourth Embodiment)

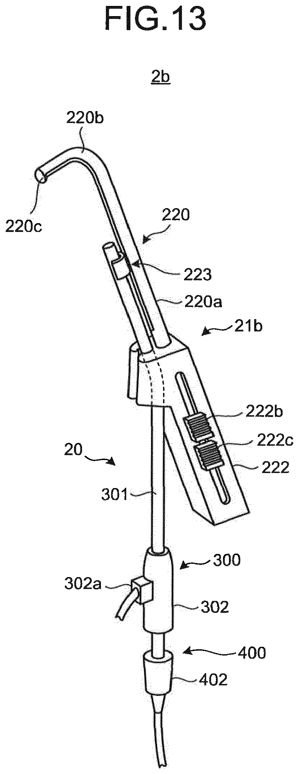

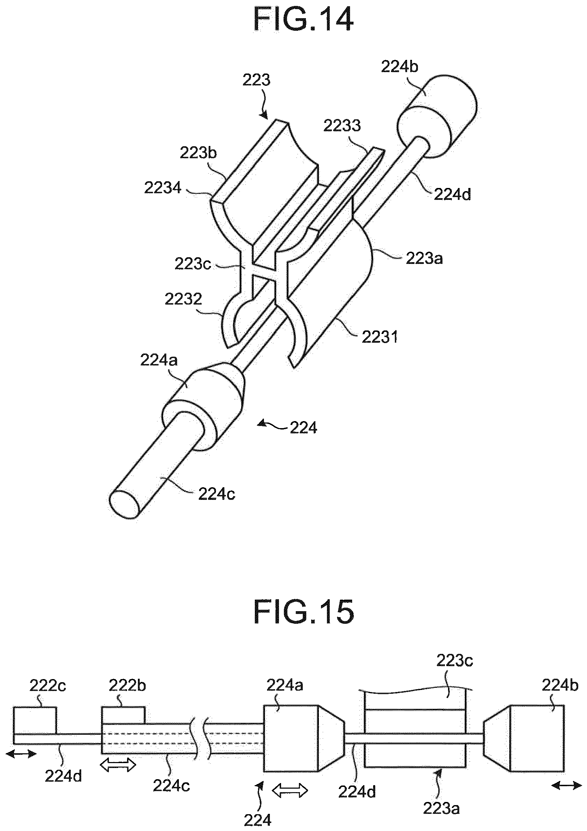

Next, a fourth embodiment of the present invention will be described. FIG. 13 is a perspective view schematically illustrating a configuration of a medical device 2b according to the fourth embodiment. FIG. 14 is a perspective view schematically illustrating configurations of main parts of an insertion aid 21b. The same reference signs are used to designate the same elements as those in the above-described embodiments. In the third embodiment described above, the holding part 221b of the regulating member 221, in a predesigned shape, holds the guide sheath 301. However, in the fourth embodiment, a state in which a guide sheath 301 is held is adjusted by expanding or contracting a hollow space formed by a holding part 223b in conjunction with a moving part 223a of a regulating member 223.

The medical device 2b includes an insertion member 20 and the insertion aid 21b described above. The insertion aid 21b includes a guiding portion 220, a grip portion 222, and the regulating member 223. The guiding portion 220 is elongated and guides a traveling direction of a sheath portion 300 of the insertion member 20. The grip portion 222 is provided at a proximal end of the guiding portion 220 and gripped by a practitioner or the like. The regulating member 223 can hold the sheath portion 300 and slide along the guiding portion 220.

As described above, the guiding portion 220 is formed by curving a tubular member having a substantially cylindrical shape cut out along a longitudinal direction. Furthermore, the regulating member 223 includes the moving part 223a, the holding part 223b, and a coupling part 223c. The moving part 223a is housed in the guiding portion 220 and movable along the guiding portion 220. The holding part 223b can hold the sheath portion 300 (guide sheath 301). The coupling part 223c couples the moving part 223a and the holding part 223b.

The coupling part 223c is elastically deformable and of a substantial H-shape in a plan view. The moving part 223a is of a substantial C-shape formed by two arms 2231 and 2232 each extending in an arc from the bottom end of the coupling part 223c. Furthermore, the holding part 223b is of a substantial C-shape formed by two arms 2233 and 2234 each extending in an arc from the top end of the coupling part 223c. Therefore, when the arms 2231 and 2232 of the moving part 223a approach each other, the arms 2233 and 2234 of the holding part 223b move away from each other. Conversely, when the arms 2231 and 2232 of the moving part 223a move away from each other, the arms 2233 and 2234 of the holding part 223b approach each other. In the fourth embodiment, the arms 2233 and 2234 of the holding part 223b are caused to approach each other by moving the arms 2231 and 2232 of the moving part 223a away from each other. Thus, a holding force for holding the guide sheath 301 is applied. As possible holding states of the holding part 223b, there are a state in which the arms 2233 and 2234 of the holding part 223b are away from each other and a state in which the arms 2233 and 2234 of the holding part 223b approach each other. Hereinafter, the former state is referred to as a release state, and the latter state is referred to as a close state.

The grip portion 222 is provided with two operation sliders that can move substantially parallel to an extending direction of a first guiding part 220a (a first slider 222b and a second slider 222c).

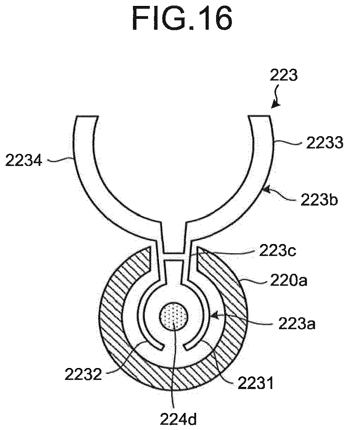

FIG. 15 is a schematic view illustrating a relationship between the slider and piece parts of the insertion aid 21b. FIG. 16 is a schematic view illustrating a holding condition of the holding part of the regulating member according to the fourth embodiment of the present invention, the view being a schematic view illustrating the first guiding part 220a and the regulating member 223 as seen along a longitudinal direction of a connecting wire 224d, in a state (release state) illustrated in FIG. 14. A state changing portion 224 (changing portion) is provided in the guiding portion 220 and the grip portion 222. The state changing portion 224 connects to the first slider 222b and the second slider 222c, and changes a holding state of the holding part 223b.

The state changing portion 224 includes a first piece part 224a, a second piece part 224b, a tubular connection sheath 224c, and the connecting wire 224d. The first piece part 224a can press the moving part 223a from one end side of the moving part 223a. The second piece part 224b can press the moving part 223a from the other end side of the moving part 223a. The connection sheath 224c connects to the first piece part 224a at one end, and connects to the first slider 222b at the other end. The connecting wire 224d is inserted into the connection sheath 224c and connects to the second piece part 224b at one end and to the second slider 222c at the other end. The first piece part 224a and the second piece part 224b each have a distal end of a tapered shape, and are of a cylindrical shape, a maximum diameter of which is larger than a diameter of the inner periphery of the arms 2231 and 2232. As illustrated in FIGS. 15 and 16, when the first piece part 224a and the second piece part 224b do not press the moving part 223a, the arms 2233 and 2234 in the holding part 223b are away from each other. Thus, the holding force is small even if the guide sheath 301 is housed in the holding part 223b.

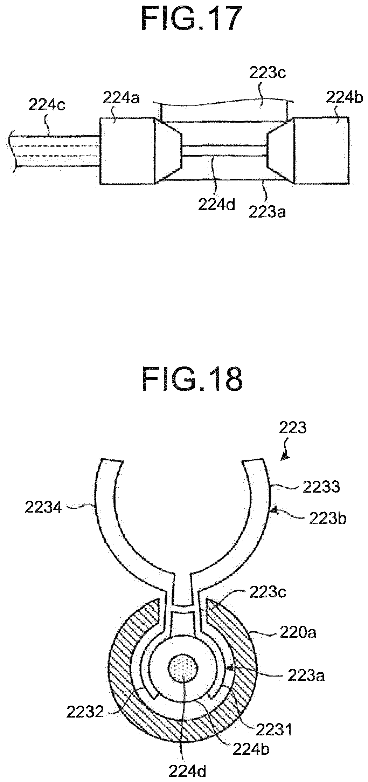

FIG. 17 is a schematic view illustrating a relationship between the moving part of the regulating member and the piece parts according to the fourth embodiment, the view illustrating a state in which the piece parts and the moving part are fitted to one another (close state). FIG. 18 is a schematic view illustrating a holding condition of the holding part of the regulating member according to the fourth embodiment, the view being a schematic view illustrating the first guiding part 220a and the regulating member 223 as seen along a longitudinal direction of the connecting wire 224d in the state illustrated in FIG. 17. When the first slider 222b and the second slider 222c are moved in a direction of approaching each other, the first piece part 224a and the second piece part 224b approach each other to press the moving part 223a, as illustrated in FIGS. 17 and 18. When the first piece part 224a and the second piece part 224b press the moving part 223a, the arms 2231 and 2232 of the moving part 223a move away from each other. Thus, the arms 2233 and 2234 of the holding part 223b approach each other. In this state, the holding part 223b holds the guide sheath 301 with a holding force larger than that in the state illustrated in FIG. 16.

As described above, when the first slider 222b and the second slider 222c are moved while the first piece part 224a and the second piece part 224b are pressing the moving part 223a, the regulating member 223 can be moved along the guiding portion 220 with a holding force applied to the guide sheath 301. When the first slider 222b and the second slider 222c are moved in a separating direction, the situation in which the first piece part 224a and the second piece part 224b press the moving part 223a is released. This decreases a holding force of holding the guide sheath 301 and thus the guide sheath 301 can be separated or slid with a smaller force.

According to the fourth embodiment of the present invention described above, the insertion aid 21b includes the guiding portion 220, the regulating member 223, the first slider 222b, and the second slider 222c. The guiding portion 220 is elongated and guides a traveling direction of the sheath portion 300 of the insertion member 20. The regulating member 223 can hold the sheath portion 300, and is housed in the guiding portion 220 and movable along the guiding portion 220. The first slider 222b and the second slider 222c operate the movement of the regulating member 223. Using the insertion aid 21b, a distal end of the guiding portion 220 is arranged in the vicinity of an opening C of a paranasal sinus. Then, the regulating member 223 holding the sheath portion 300 is slid along the guiding portion 220. Thus, the distal end of the sheath portion 300 is arranged in the vicinity of the opening C of the paranasal sinus. With this configuration, the insertion member 20 can be surely guided regardless of the minimum bend radius defined by the hard length of a distal end rigid part 401a of an insertion portion 401.

Furthermore, in the fourth embodiment described above, there is further provided the state changing portion 224 that changes a holding state of the holding part 223b. Thus, the holding state of the holding part 223b can be changed by the operation of the first slider 222b and the second slider 222c. With this configuration, the holding force of holding the guide sheath 301 can be adjusted, and the guide sheath 301 (insertion member 20) can be guided more surely.

### (Fifth Embodiment)

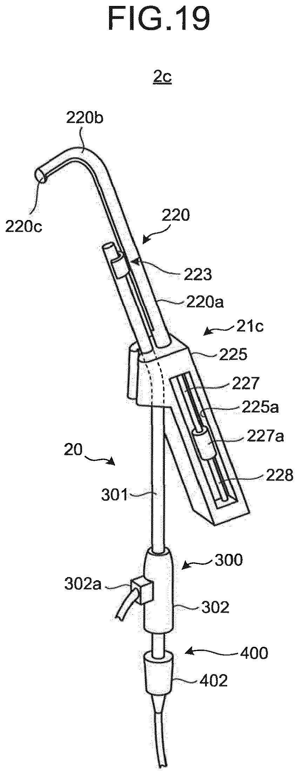

Next, a fifth embodiment of the present invention will be described. FIG. 19 is a perspective view schematically illustrating a configuration of a medical device 2c according to the fifth embodiment. The same reference signs are used to designate the same elements as those in the above-described embodiments. In the fourth embodiment described above, the hollow space formed by the holding part 223b is expanded or contracted using the two piece parts. However, in the present embodiment, a hollow space formed by a holding part 223b is expanded or contracted using a flat member.

As illustrated in FIG. 19, the medical device 2c includes an insertion member 20 and an insertion aid 21c described above. The insertion aid 21c includes a guiding portion 220, a grip portion 225, and a regulating member 223. The guiding portion 220 is elongated and guides a traveling direction of a sheath portion 300 of the insertion member 20. The grip portion 225 is provided at a proximal end of the guiding portion 220 and gripped by a practitioner or the like. The regulating member 223 can hold the sheath portion 300 and slide along the guiding portion 220.

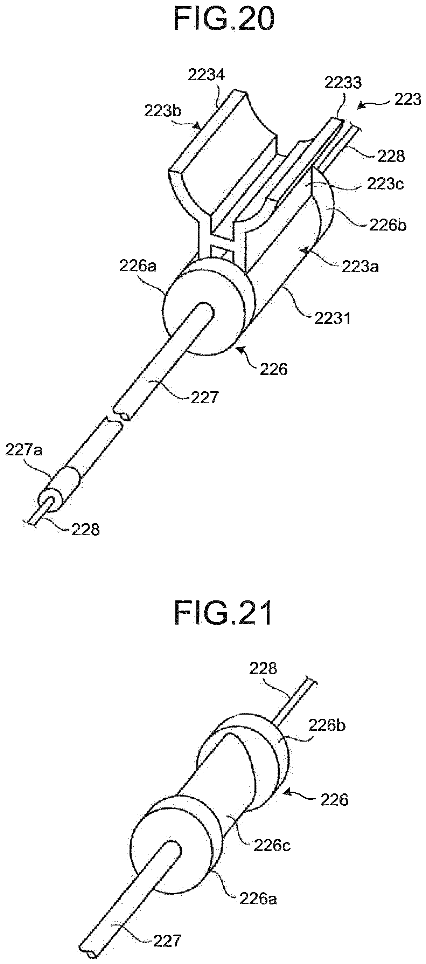

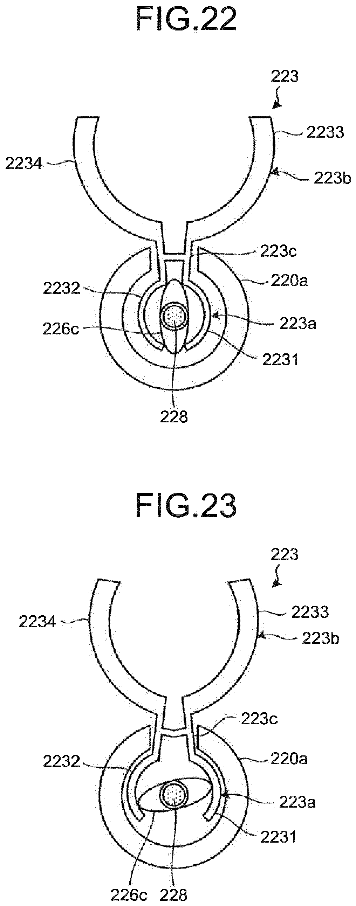

FIG. 20 is a perspective view schematically illustrating configurations of main parts of the insertion aid 21c. FIG. 21 is a schematic view illustrating piece parts of the insertion aid 21c. FIG. 22 is a schematic view illustrating a holding condition of the holding part of the regulating member according to the fifth embodiment of the present invention, the view being a schematic view illustrating the first guiding part 220a and the regulating member 223 as seen along a longitudinal direction of an operation pipe 227, in a release state. A state changing portion 226 (changing portion), the tubular operation pipe 227, and a guide wire 228 are provided in the guiding portion 220 and the grip portion 225. The state changing portion 226 changes a holding state of the holding part 223b. The operation pipe 227 extends from the state changing portion 226 to the grip portion 225. The guide wire 228 is inserted into the state changing portion 226 and the operation pipe 227, connects a distal end of the guiding portion 220 and a proximal end of the grip portion 225, and guides the movement of the state changing portion 226 and the operation pipe 227. The grip portion 225 has an opening part 225a exposing a part of the operation pipe 227 to the outside. The guide wire 228 may not be provided as long as the state changing portion 226 and the operation pipe 227 are movable together along the guiding portion 220.

The state changing portion 226 is discoid and includes two engaging parts (a first engaging part 226a and a second engaging part 226b) and a piece part 226c. The engaging parts can engage with ends of the moving part 223a. The piece part 226c is provided inside the moving part 223a and pressed against or spaced from an inner wall of the moving part 223a.

The first engaging part 226a and the second engaging part 226b are provided at both ends of the moving part 223a. The piece part 226c has an elliptical cross section cut along a plane perpendicular to a direction in which the first engaging part 226a and the second engaging part 226b face each other (extending direction of the piece part 226c). In the piece part 226c, a long axis of the cross section is longer than the maximum distance between the inner faces of arms 2231 and 2232, and a short axis of the cross section is shorter than the maximum distance between the arms 2231 and 2232.

On end of the operation pipe 227 is coupled to the first engaging part 226a and the other end of the operation pipe 227 is coupled to the proximal end of the grip portion 225. Therefore, when the operation pipe 227 rotates around a longitudinal axis, the state changing portion 226 rotates in conjunction with the rotation of the operation pipe 227. A straight line extending along the longitudinal axis of the operation pipe 227 and passing through the longitudinal axis preferably passes through each center of the first engaging part 226a, the second engaging part 226b, and the piece part 226c. Furthermore, the first engaging part 226a, the second engaging part 226b, the piece part 226c, and the operation pipe 227 are integrally movable along the guide wire 228.

Furthermore, a circular ring part 227a that is used by the practitioner to operate the rotation of the operation pipe 227 is provided at an end of the operation pipe 227 that is a part exposed to the outside through the opening part 225a of the operation pipe 227. The surface of the circular ring part 227a is preferably subjected to knurling processing or the like from a viewpoint of operability.

As illustrated in FIG. 22, in a case where the holding part 223b is in the release state, short-axis sides in a cross section of the piece part 226c face the arms 2231 and 2232, and the piece part 226c does not abut on the arms 2231 and 2232 of the moving part 223a.

FIG. 23 is a schematic view illustrating a holding condition of the holding part of the regulating member according to the fifth embodiment of the present invention, the view being a schematic view illustrating the first guiding part 220a and the regulating member 223 as seen along the longitudinal direction of the operation pipe 227, in a close state. When the operation pipe 227 rotates around the longitudinal axis, the state changing portion 226 rotates in conjunction with the rotation of the operation pipe 227. For example, when the operation pipe 227 is rotated by 90 degrees in the state illustrated in FIG. 22, long-axis sides in the cross section of the piece part 226c face the arms 2231 and 2232, and the piece part 226c presses against (presses in) the arms 2231 and 2232 of the moving part 223a, as illustrated in FIG. 23. In a case where the long-axis sides of the piece part 226c abut on the arms 2231 and 2232 of the moving part 223a, the arms 2231 and 2232 move away from each other. Thus, the arms 2233 and 2234 of the holding part 223b approach each other. In this state, the holding part 223b holds the guide sheath 301 with a holding force larger than that in the state illustrated in FIG. 22.

As described above, when the first engaging part 226a, the second engaging part 226b, the piece part 226c, and the operation pipe 227 are moved along the guide wire 228 while the long-axis sides of the piece part 226c abut on the arms 2231 and 2232 of the moving part 223a, the regulating member 223 can be moved along the guiding portion 220 with a holding force applied to the guide sheath 301. Furthermore, when the operation pipe 227 rotates, contact between the piece part 226c and the moving part 223a is released. This decreases a holding force of the guide sheath 301 and thus the guide sheath 301 can be separated or slid with a smaller force.

According to the fifth embodiment of the present invention described above, the insertion aid 21c includes the guiding portion 220, the regulating member 223, the state changing portion 226, the operation pipe 227, and the guide wire 228. The guiding portion 220 is elongated and guides a traveling direction of the sheath portion 300 of the insertion member 20. The regulating member 223 can hold the sheath portion 300, and is housed in the guiding portion 220 and slidable along the guiding portion 220. The state changing portion 226 changes a holding state of the holding part 223b. The operation pipe 227 and the guide wire 228 operate the movement of the regulating member 223. Using the insertion aid 21c, a distal end of the guiding portion 220 is arranged in the vicinity of an opening C of a paranasal sinus. Then, the regulating member 223 holding the sheath portion 300 is slid along the guiding portion 220. Thus, a distal end of the sheath portion 300 is arranged in the vicinity of the opening C of the paranasal sinus. With this configuration, the insertion member 20 can be surely guided regardless of the minimum bend radius defined by the hard length of a distal end rigid part 401a of an insertion portion 401.

Furthermore, in the fifth embodiment described above, there are further provided the state changing portion 226 and the operation pipe 227. The state changing portion 226 changes a holding state of the holding part 223b, and includes the piece part 226c. The operation pipe 227 rotationally operates the piece part 226c. Thus, the holding state of the holding part 223b can be changed by the rotation of the piece part 226c through a rotational operation of the operation pipe 227. With this configuration, the holding force of holding the guide sheath 301 can be adjusted, and the guide sheath 301 (insertion member 20) can be guided more surely.

### (Sixth Embodiment)

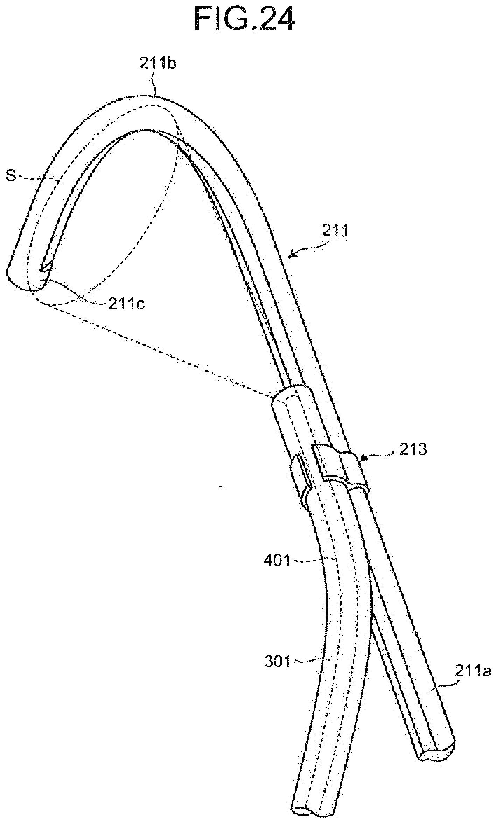

Next, a sixth embodiment of the present invention will be described. FIG. 24 is a schematic view illustrating an exemplary arrangement of an insertion member according to the sixth embodiment of the present invention. The same reference signs are used to designate the same elements as those in the above-described embodiments. According to the sixth embodiment, in the first embodiment described above, a practitioner inserts a distal end of a guiding portion 211 into an opening while checking an image including the distal end of the guiding portion 211 displayed on a monitor 5 in a state in which the distal end of the guiding portion 211 is put in a field of view of an insertion member 20 (endoscope 400).

As illustrated in FIG. 24, the positions of a regulating member 213 and the insertion member 20 are adjusted such that the distal end (convex part 211c) of the guiding portion 211 is in a field of view S of the insertion member 20. Thus, an insertion aid 21 can be inserted into a nasal cavity while the distal end of the guiding portion 211 is checked. The positions of the regulating member 213 and the insertion member 20 can be adjusted using a marker marked on the guiding portion 211 or the like, a click mechanism that positions the regulating member 213 and the insertion member 20, or the like.

### (Seventh Embodiment)

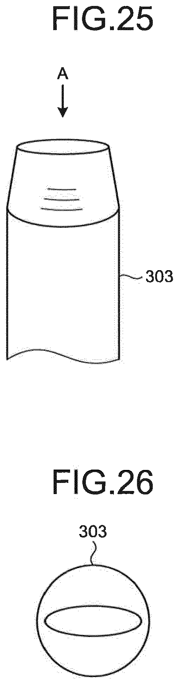

Next, a seventh embodiment of the present invention will be described. FIG. 25 is a schematic view illustrating an exemplary configuration of a distal end of a guide sheath according to the seventh embodiment of the present invention. FIG. 26 is a top view as seen from an arrow A direction illustrated in FIG. 25. The same reference signs are used to designate the same elements as those in the above-described embodiments. In the first to sixth embodiments described above, the guide sheath 301 has a cylindrical shape. However, a guide sheath 303 according to the seventh embodiment has elasticity and a shape in which a distal end thereof has a reduced diameter.

As illustrated in FIGS. 25 and 26, a distal end part of a distal end rigid part 401a has a reduced diameter. With this reduced diameter, for example, even in a case where a hole section of an opening C (for example, refer to FIG. 5) becomes small because of pus or the like, the guide sheath 303 is caused to easily access the opening C, and a part of the distal end, for example, is inserted into the opening C. Thus, the insertion member 20 can be easily inserted into the opening C. Instead of the configurations illustrated in FIGS. 25 and 26, the distal end may have a gradually reduced diameter.

The present invention is not limited to the first to seventh embodiments described above. The present invention can include various embodiments within the scope of technical ideas described in claims.

### Industrial Applicability

As described above, an insertion aid and a medical device according to the present invention are useful in surely guiding an insertion member into a paranasal sinus or an opening leading to the paranasal sinus even if the insertion member has a rigid distal end. Reference Signs List

1 Paranasal sinuses observation system
2 Medical device
3 Controller
4 Liquid supply and suction operating unit
5 Monitor
20 Insertion member
21 Insertion aid
211, 214, 216, 218, 220 Guiding portion
212, 222, 225 Grip portion (support portion)
213, 215, 217, 219, 221, 223 Regulating member (guided portion)
224, 226 State changing portion
300 Sheath portion
301, 303 Guide sheath
400 Endoscope
401 Insertion portion
401a Distal end rigid part
401b Flexible tube part

## Claims

1. An insertion aid for assisting in insertion of an insertion member having a rigid distal end part into a paranasal sinus or an opening leading to the paranasal sinus, the insertion aid comprising:
a guiding portion having an elongated and curved one end part and configured to guide an insertion route of the insertion member;
a guided portion including a holding part for holding the insertion member and including a moving part continuous to the holding part and movable along the guiding portion; and
a support portion supporting the other end part of the guiding portion.

2. The insertion aid according to claim 1, further comprising a rotation preventing portion configured to prevent the guided portion from rotating around a longitudinal axis of the guiding portion.

3. The insertion aid according to claim 1, wherein
the guiding portion has a D-shaped cross-section perpendicular to a longitudinal axis of the guiding portion, and
the moving part includes an insertion through hole which has a D-shaped cross-section and through which the guiding portion is inserted.

4. The insertion aid according to claim 1, wherein
the guiding portion has a cylindrical shape with a slit along a longitudinal axis of the guiding portion, and
the guided portion includes a coupling part coupling the holding part and the moving part and engaging with the slit.

5. The insertion aid according to claim 1, further comprising:
a slider provided on the support portion and movable in a direction substantially parallel to an extending direction of the guiding portion extending from the support portion; and
a connecting member connecting the slider and the guided portion.

6. The insertion aid according to claim 1, wherein
holding force for holding the insertion member by the holding part varies depending on a load applied to the moving part, and
the insertion aid further comprises a changing portion configured to change the holding force.

7. The insertion aid according to claim 6, wherein
the changing portion comprises:
a press-fit member configured to press the moving part; and
an operation member configured to operate the press-fit member.

8. A medical device comprising:
an insertion member having a rigid distal end part; and
an insertion aid for assisting in insertion of the insertion member into a paranasal sinus or an opening leading to the paranasal sinus, the insertion aid comprising:
a guiding portion having an elongated and curved one end part and configured to guide an insertion route of the insertion member;
a guided portion including a holding part for holding the insertion member and including a moving part continuous to the holding part and movable along the guiding portion; and
a support portion supporting the other end part of the guiding portion.
